# EUROPEAN PATENT SPECIFICATION

(11) **EP 3 578 550 B1**
(45) Date of publication and mention of the grant of the patent: **16.08.2023**
(21) Application number: 19175963.8
(22) Date of filing: 22.05.2019
(51) Int. Cl.: C07D 249/20, G03C 1/815, C09D 7/48

(54) **NOVEL RED-SHIFTING BENZOTRIAZOLE UV ABSORBERS**
NEUARTIGE BENZOTRIAZOL-UV-ABSORBER MIT ROTVERSCHIEBUNG
NOUVEAUX ABSORBEURS D'UV DE TYPE BENZOTRIAZOLE À DÉCALAGE ROUGE

(30) Priority: 05.06.2018 TW 107119280; 19.03.2019 TW 108109341
(43) Date of publication of application: 11.12.2019
(73) Proprietor: Everlight Chemical Industrial Corporation, 106 Taipei City (TW)
(72) Inventor: CHEN, Chi-Ren, 328 Taoyuan City (TW); LIN, Jing-Wen, 328 Taoyuan City (TW); YEN, Meng-Huang, 328 Taoyuan City (TW); HUANG, Yao-Hsing, 328 Taoyuan City (TW); CHEN, Ko-Lun, 328 Taoyuan City (TW); CHEN, Chih-Wei, 328 Taoyuan City (TW)
(74) Representative: van Trier, Norbertus Henricus Gerardus

(56) References cited:
- WO-A1-2010/053917
- JP-A- 2008 105 958
- US-A- 3 738 837
- US-A- 5 278 314
- US-A1- 2002 065 341
- Joanna Paluszkiewicz ET AL: "Reactive Dyes for Cellulose Fibres Including UV Absorbers", , 1 June 2005 (2005-06-01), XP055610850, Retrieved from the Internet: URL:http://www.fibtex.lodz.pl/50_20_76.pdf [retrieved on 2019-08-05]

## Description

### CROSS REFERENCE TO RELATED APPLICATION

This application claims the benefits of the Taiwan Patent Application Serial Numbers 107119280 and 108109341, respectively filed on June 5, 2018 and March 19, 2019.

### BACKGROUND

### 1. Field

The present disclosure provides a novel benzotriazole UV absorber with red shift and a use thereof. More specifically, the present disclosure provides a novel benzotriazole UV absorber having excellent UV absorption capacity and a composition, a glasses lens and a screen protector containing the same.

### 2. Description of Related Art

The UV absorber is a light stabilizer, which can absorb the UV light in the sunlight or in the fluorescent light source to protect the substance added with the UV absorber against the damage from the UV light. Alternatively, when a coating layer containing the UV absorber is formed on a substrate, the coating layer can also protect the substrate against the damage from the UV light.

Nowadays, the known UV absorbers can be mainly classified into benzotriazole UV absorbers, salicylate UV absorbers, benzophenone UV absorbers, substituted acrylonitrile UV absorbers, triazine UV absorbers and hindered amines UV absorbers. Among these UV absorbers, the benzotriazole UV absorbers are UV absorbers with high stability, which can effectively protect the substance or the substrate containing the benzotriazole UV absorbers against the damage caused by the UV light.

However, the commercially available benzotriazole UV absorbers still have their absorption limit, which is about 400 nm.

Typical benzotriazole UV absorbers are disclosed in documents US2002/065341, US5278314 and WO2010/053917. Thus, it is desirable to provide a novel benzotriazole UV absorber, wherein the absorption spectrum thereof has red shift; therefore, the application of the benzotriazole UV absorber can be extended.

### SUMMARY

An object of the present disclosure is to provide a novel compound which has excellent UV absorption capacity.

The novel compound provided by the present disclosure has a structure represented by the following formula (I): 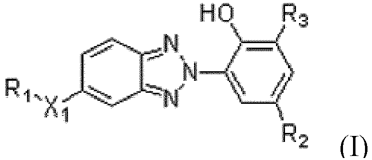
wherein X₁ is -S-, -SO-, or -SO₂-;
R₁ is C₁₋₂₄ linear or branched alkyl, or C₆₋₁₅ aryl;
R₂ is -OR₄ or -NR₅R₆, wherein R₄, R₅ and R₆ respectively are H, C₁₋₁₀ linear or branched alkyl, C₇₋₁₅ phenylalkyl, or C₁₋₁₀ linear or branched alkyl substituted by one or more -OH, or -NR₁₁R₁₂; R₁₁ is H or C₁₋₁₀ linear or branched alkyl; R₁₂ is H, C₁₋₁₀ linear or branched alkyl or -C(=O)R₁₈, wherein R₁₈ is linear or branched C₂₋₁₀ alkenyl, -OR₁₉ or -NR₂₀R₂₁, R₁₉ is C₁₋₁₀ linear or branched alkyl or C₆₋₁₅ aryl, R₂₀ and R₂₁ respectively are H, C₁₋₁₀ linear or branched alkyl or C₆₋₁₅ aryl; and
R₃ is H, C₁₋₁₀ linear or branched alkyl,
when R₂ is -OR₄, R₄ is H, C₁₋₁₀ linear or branched alkyl, C₇₋₁₅ phenylalkyl or C₁₋₁₀ linear or branched alkyl substituted by one or more -OH or -NR₁₁R₁₂ and R₃ is C₁₋₁₀ linear or branched alkyl; and
when R₂ is -NR₅R₆, R₅ and R₆ respectively are H or C₁₋₁₀ linear or branched alkyl and R₃ is H.

The novel compound provided by the present disclosure is a benzotriazole compound, which can be used as an UV absorber. The X₁ group on the benzo ring of the formula (I) is an S-containing group. The R₂ group on the phenyl ring of the formula (I) is an O-containing group or an N-containing group. The collaboration between the X₁ group and the R₂ group makes the compound have red shift in the absorption spectrum thereof, and thus the compound has a broader absorption range. In particular, the novel compound provided by the present disclosure has high absorption in the long wavelength region from 380 nm to 430 nm, and the maximum absorption wavelength is 382 nm. In addition, the novel compound provided by the present disclosure has broader absorption range, and the absorption range can be extended to 450 nm or more. Furthermore, the novel compound of the present disclosure also has the absorption peaks that the current UV absorbers have in the region from 290 nm to 340 nm. Hence, the novel compound provided by the present disclosure can be applied to anti-blue light materials, such as the glasses lens or the contact lens, and further can be applied to carbon fiber composites used in the aerospace or vehicle industry.

In one aspect of the present disclosure, X₁ is -S-, -SO-, or -SO₂-. Preferably, X₁ is -S- or -SO₂.

In one aspect of the present disclosure, R₁ is C₁₋₂₄ linear or branched alkyl, or C₆₋₁₅ aryl. Preferably, R₁ is C₁₋₁₅ linear or branched alkyl, C₆₋₁₅ aryl. Most preferably, R₁ is C₁₋₁₅ linear or branched alkyl, phenyl or naphthyl. In one example of the present disclosure, R₁ is phenyl or dodecyl, but the present disclosure is not limited thereto.

In one aspect of the present disclosure, R₂ is -OR₄ or -NR₅R₆, wherein R₄, R₅ and R₆ respectively are H, C₁₋₁₀ linear or branched alkyl, C₇₋₁₅ phenylalkyl, or C₁₋₁₀ linear or branched alkyl substituted by one or more -OH, or -NR₁₁R₁₂; R₁₁ is H or C₁₋₁₀ linear or branched alkyl; R₁₂ is H, C₁₋₁₀ linear or branched alkyl or -C(=O)R₁₈, wherein R₁₈ is linear or branched C₂₋₁₀ alkenyl, -OR₁₉ or -NR₂₀R₂₁, R₁₉ is C₁₋₁₀ linear or branched alkyl or C₆₋₁₅ aryl, R₂₀ and R₂₁ respectively are H, C₁₋₁₀ linear or branched alkyl or C₆₋₁₅ aryl.

In one aspect of the present disclosure, R₂ can be -OR₄, in which case R₄ is H, C₁₋₁₀ linear or branched alkyl, C₇₋₁₅ phenylalkyl or C₁₋₁₀ linear or branched alkyl substituted by one or more -OH, or -NR₁₁R₁₂; R₁₁ is H or C₁₋₁₀ linear or branched alkyl; R₁₂ is H, C₁₋₁₀ linear or branched alkyl or -C(=O)R₁₈, wherein R₁₈ is linear or branched C₂₋₁₀ alkenyl, -OR₁₉ or -NR₂₀R₂₁, R₁₉ is C₁₋₁₀ linear or branched alkyl or C₆₋₁₅ aryl, R₂₀ and R₂₁ respectively are H, C₁₋₁₀ linear or branched alkyl or C₆₋₁₅ aryl. Preferably, R₂ is -OR₄, and R₄ is H, C₁₋₁₀ linear or branched alkyl, C₇₋₁₅ phenylalkyl, or C₁₋₁₀ linear or branched alkyl substituted by one or more -OH or -NR₁₁R₁₂; wherein R₁₁ and R₁₂ respectively are H or C₁₋₁₀ linear or branched alkyl. In one example of the present disclosure, R₂ is -OR₄, and R₄ is H, benzyl, methyl, ethyl, propyl, butyl, or methyl, ethyl or propyl substituted with one -OH, dimethylamino group, diethylamino group, dipropylamino group or dibutylamino group; but the present disclosure is not limited thereto.

In one aspect of the present disclosure, R₂ can be -NR₅R₆, in which case R₅ and R₆ respectively are H, C₁₋₁₀ linear or branched alkyl. More preferably, R₂ is -NR₅R₆, and R₅ and R₆ are the same and are H or C₁₋₁₀ linear or branched alkyl. In one example of the present disclosure, R₂ is -NR₅R₆, and R₅ and R₆ are the same and are H, methyl, ethyl, propyl or butyl; but the present disclosure is not limited thereto.

In one aspect of the present disclosure, R₃ is H, C₁₋₁₀ linear or branched alkyl. Most preferably, R₃ is H or C₁₋₆ linear or branched alkyl. In one example of the present disclosure, R₃ is H or tert-butyl; but the present disclosure is not limited thereto.

In one aspect of the present disclosure, the compound of the formula (I) can be anyone represented by the following formulas (I-1) to (I-10):

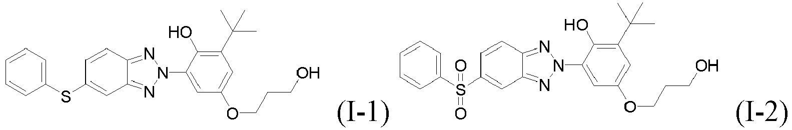

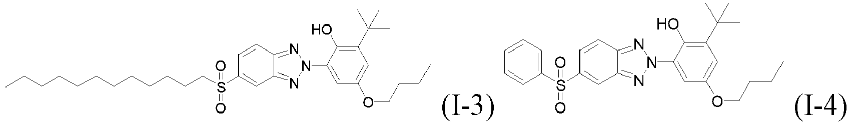

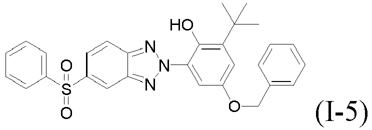

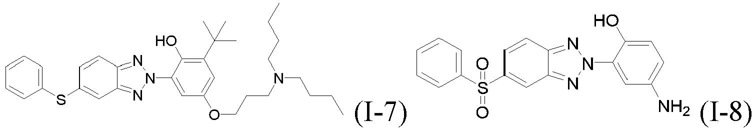

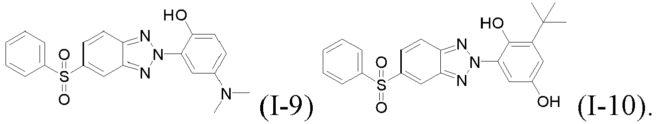

In the present disclosure, the term "alkyl" includes linear or branched alkyl, and includes, for example, C₁₋₂₄ alkyl, C₁₋₁₅ alkyl, C₁₋₁₀ alkyl, C₁₋₈ alkyl or C₁₋₆ alkyl. Examples of the alkyl include, but are not limited to: alkyl, ethyl, n-propyl, iso-propyl, n-butyl, iso-butyl, sec-butyl, tert-butyl, pentyl, neo-pentyl and hexyl.

In the present disclosure, the term "alkenyl" includes linear or branch hydrocarbon groups with at least one double bond, and includes, for example, linear or branch C₂₋₁₈ hydrocarbon groups with at least one double bond, linear or branch C₂₋₁₀ hydrocarbon groups with at least one double bond, linear or branch C₂₋₈ hydrocarbon groups with at least one double bond or linear or branch C₂₋₆ hydrocarbon groups with at least one double bond. Examples of the alkenyl include, but are not limited to vinyl, propenyl and butenyl.

In the present disclosure, the term "cycloalkyl" includes monovalent or bivalent cyclic saturated hydrocarbon groups, which includes, for example, 5 to 12 carbon atoms (C₅₋₁₂), 5 to 10 carbon atoms (C₅₋₁₀) or 5 to 8 carbon atoms (C₅₋₈). Examples of the cycloalkyl include, but are not limited to cyclopentyl, cyclohexyl, 1,4-cyclohexyl, cycleheptyl, cyclooctyl and adamantine.

In the present disclosure, the term "aryl" includes 6-membered single aromatic ring, 10-membered double aromatic ring or 14-membered triple aromatic ring. Examples of the aryl include, but are not limited to phenyl, naphthyl, pyrenyl, anthryl and phenanthryl.

In the present disclosure, the term "phenylalkyl" refers to a group that the alkyl defined in the present disclosure is added with one phenyl group. Examples of the phenylalkyl include, but are not limited to methylphenyl (benzyl), ethylphenyl and propylphenyl.

In the present disclosure, the term "alkali metal" includes Li, Na or K.

In addition, the present disclosure further provides a composition stabilized against light-induced degradation, which comprises: (A) an organic material subjected to light-induced degradation; and (B) the aforesaid novel compound of the present disclosure. Herein, a content of the compound can be ranged from 0.1 wt% to 30 wt% based on a total weight of the organic compound.

In one aspect of the present disclosure, the aforesaid composition is used to form a coating layer. In particular, the coating layer is formed on a substrate, and the substrate is sensitive to an electro-magnetic radiation having wavelengths greater than 380 nm. Herein, the material of the substrate is not particularly limited, and can be glass, plastic, polymer, silicone hydrogel, resin, carbon fiber composites or a combination thereof.

Furthermore, the present disclosure further provides a lens for a blue light blocking glasses, which comprises the aforesaid novel compound of the present disclosure. Herein, a substrate for forming the lens is coated with the novel compound of the present disclosure to form an anti-UV coating layer or a blue light blocking coating layer.

In addition, the present disclosure further provides an anti-blue light screen protector, which comprises the aforesaid novel compound of the present disclosure. Herein, a substrate for forming the screen protector is coated with the novel compound of the present disclosure to form an anti-UV coating layer or a blue light blocking coating layer.

Other objects, advantages, or novel features of the disclosure will become more apparent from the following detailed description.

### DETAILED DESCRIPTION OF EMBODIMENT

The following embodiments when read with the accompanying drawings are made to clearly exhibit the above-mentioned and other technical contents, features and/or effects of the present disclosure. Through the exposition by means of the specific embodiments, people would further understand the technical means and effects the present disclosure adopts to achieve the above-indicated objectives.

Unless specified otherwise, singular words "a" and "the" used in the present specification and claims include one or plural objects.

Unless specified otherwise, singular words "a" and "the" used in the present specification and claims include one or plural objects.

The present disclosure is explained by the following examples, which are not used to limit the scope of the present disclosure. Unless specified otherwise, in the following examples and comparative examples, the unit "%" used to represent the content or the amount of the component is based on the weight of the component.

### Synthesis of raw material Compound (1a)

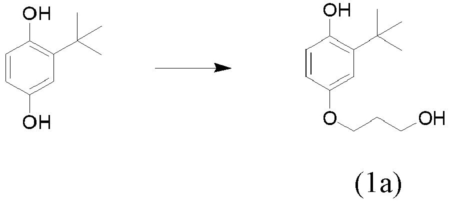

856.8 g of tertiary butylhydroquinone was dissolved in 2.5 L of methanol, followed by adding 206.2 g of NaOH, 1L of water, 482.3 g of chloropropanol and 6.3 g of KI. The mixture was heated at a reflux temperature and under N₂ atomosphere for 48 hr. After the mixture was cooled down to room temperature, 5.6 L of water was added to dilute the mixture. The diluted mixture was extracted with 4 L of dichloromethane, and the combined dichloromethane layer was washed with water, dried with anhydrous sodium sulfate and concentrated. The residue was distilled under reduced pressure, and the distillate was further purified by recrystallization with toluene to obtain 396.0 g of white solids (mp = 78.5-80.5°C).

### Synthesis of Compound (1b)

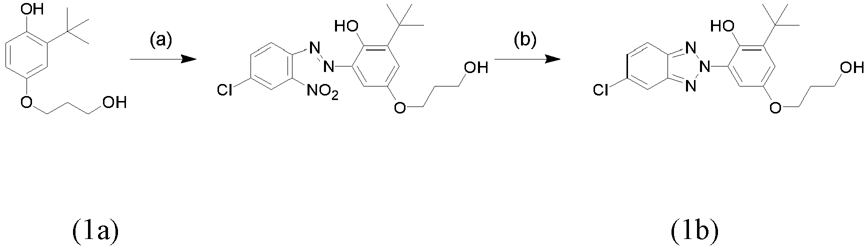

### Step (a)

90.6 g of 4-chloro-2-nitroaniline and 180 mL concentrated HCl was stirred at room temperature for 1 hr, followed by dilluting with 160 mL of water and 300 g of ice. At -5°C, 37.7 g of NaNO₂ (dissolved in 140 mL of water) was added dropwisely into the mixture, followed by stirring at 0°C for 1 hr. Sulfamic acid was added into the mixture until the result of the potassium iodide-starch test indicated negative. Then, the mixture was filtered, and the filtrate was added dropwisely into a mixing solution containng 112.2 g of Compound (1a), 60 g of NaOH and 2 L of water at -5°C to 0°C. After adding about 1/3 of an diazonium solution, 400 mL of NaOH solution (10%) was added dropwisely and together with the diazonium solution into the reaction mixture, and the addings of the diazonium solution and the NaOH solution were finished at the same time. During the dropwise adding step, the temperature of the reaction mixture was kept below 0°C. After the dropwise adding was finished, the reaction mixture was kept at the same temperature and stirred for 2 hr, and then the temperature of the reaction mixture was back to room temperature. The azo dye was separated by acidification with HCl, followed by filtration and washing with water. The azo dye was directly used in the next step without purification.

### Step (b)

The azo dye obtained in the step (a) was dissolved in 1.5 L of ethanol. A glucose solution (180 g of glucose dissolved in 1.5 L of NaOH aqueous solution (2N)) was added slowly into the reaction solution, and the temperature of the reaction solution was kept below 30°C. A thin layer chromotagraphy was held to trace the reaction until the reaction was completed. Next, 165 g of active zinc powder was added. After the mixture was stirred at room temperature for 3 hr, the mixture was dilluted with 1 L of water and stirred for 15 hr. After standing for 1 hr, the mixture was filtered, and the obtained precipitate was washed with water. The filtration cake was then extrated with 2 L of hot ethanol until only zinc was remained in the filtration cake. The extract was cooled down to room temperature, and the solid was filtered out and washed with cold ethanol. After vaccum drying, 67.8 g of yellow solids were obtained (mp = 134.7°C).
¹H NMR (400MHz, CDCl₃): δ 11.35 (1H), 7.92 (1H), 7.87 (1H), 7.80 (1H), 7.43 (1H), 7.02 (1H), 4.20 (2H), 3.91 (2H), 2.10 (2H), 1.80 (1H), 1.49 (9H)
TGA (5% weight loss): 246.23°C

### Synthesis of Compound (1c) (Example 1, formula (I-1))

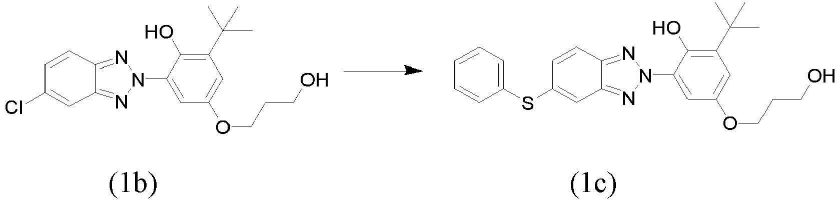

5.9 g of Compound (1b) was dissolved in 10.5 g of N-methylpyrrolidone. At 90 °C, 4.43 g of KOH aqueous solution (45 %) was added slowly into the solution, followed by dropwisely adding 2.04 g of thiophenol. Then, the solution was heated to 170°C. After water removing for 24 hr, the solution was cooled down to 100°C. The solution was extracted with 75 mL of xylene, followed by washing with 75 mL of water. After the water layer was acidified with 15% of HCl, the water layer was removed, and the organic layer was dried with anhydrous sodium sulfate and concentrated. After standing, 2.58 g of yellow solids was precipitated (mp = 128.3°C).
HRMS ESI [M+H]⁺: calc. 450.1851, found 450.1850
¹H NMR (400MHz, DMSO-d6): δ 10.79 (1H), 8.07 (1H), 7.85 (1H), 7.55 (1H), 7.55-7.40 (6H), 6.95 (1H), 4.56 (1H), 4.05 (2H), 3.57 (2H), 1.88 (2H), 1.43 (9H)
TGA (5% weight loss): 310.77°C

### Synthesis of Compound (1d) (Example 2, formula (1-2))

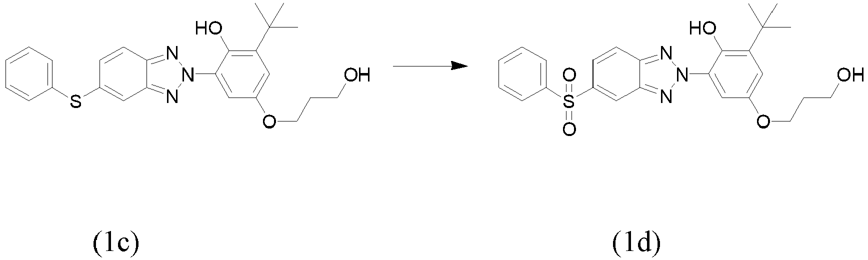

2.58 g of Compound (1c) was dissolved in 4.28 g of xylene, followed by adding 0.038 g of Na₂WO₄ and 0.85 of formic acid (90%). The solution was heated to 50°C, followed by dropwisely adding 2.54 g of hydrogen peroxide (30%) therein. The reaction temperature during adding the hydrogen peroxide was not exceeded 85°C, and the thin layer chromotagraphy was held to trace the reaction until the reaction was completed. Then, xylene and water was added, followed by extraction, drying and concentration. After recrystallized in methanol, 1.98 g of yellow solids were obtained (mp = 125.1°C).
HRMS ESI [M+H]⁺: calc. 482.1750, found 482.1748
¹H NMR (400MHz, CDCl₃): δ 11.24 (1H), 8.71 (1H), 7.95-8.10 (3H), 7.90 (1H), 7.81 (1H), 7.50-7.70 (3H), 7.06 (1H), 4.21 (2H), 3.91 (2H), 2.10 (2H), 1.80 (1H), 1.48 (9H)
TGA (5% weight loss): 341.01°C

### Synthesis of raw material Compound (2a)

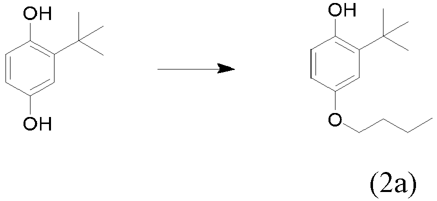

The synthesis method of Compound (2a) is similar to that of Compound (1a). The starting material used herein was 856.8 g of tertiary butylhydroquinone and 706.3 g of bromobutane, and 590.1 g of light yellow liquid was obtinaed.

### Synthesis of Compound (2b)

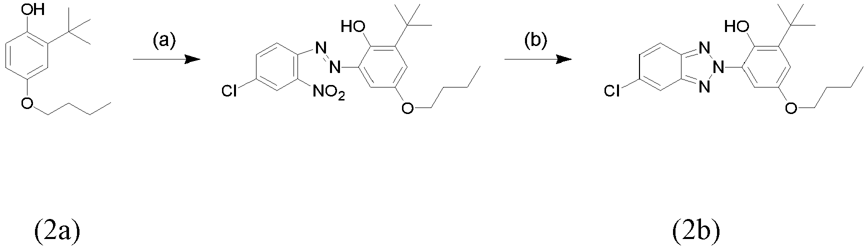

The synthesis method of Compound (2b) is similar to that of Compound (1b). The starting material used herein was 450.0 g of the raw material Compound (2a), and 159.6 g of yellow solids were obtained (mp = 101.6°C).
HRMS ESI [M-H]⁻: calc. 372.1479, found 372.1477
¹H NMR (400MHz, CDCl₃): δ 11.31 (1H), 7.91 (1H), 7.85 (1H), 7.75 (1H), 7.41 (1H), 7.02 (1H), 4.03 (2H), 1.81 (2H), 1.54 (2H), 1.49 (9H), 1.00 (3H)

### Synthesis of Compound (2d) (Example 3, formula (1-3))

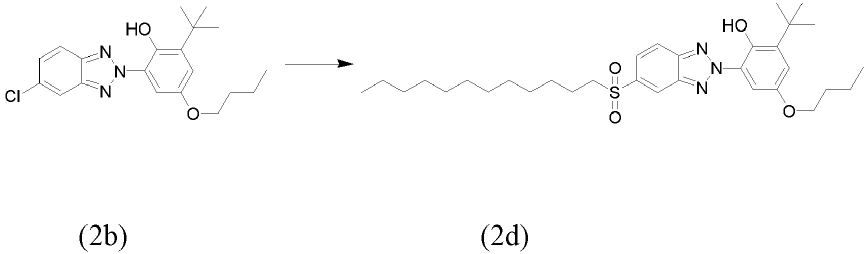

The synthesis method of Compound (2d) is similar to those of Compound (1c) and Compound (1d). The starting material used herein was 40.0 g of Compound (2b) and 200 mL of 1-dodecanethiol, and 40.6 g of yellow liquid was obtained.
HRMS ESI [M+H]⁺: calc. 572.3522, found 572.3531
¹H NMR (400MHz, CDCl₃): δ 11.24 (1H), 8.63 (1H), 8.11 (1H), 7.92 (1H), 7.81 (1H), 7.07 (1H), 4.05 (2H), 3.18 (2H), 1.70-1.90 (4H), 1.10-1.70 (20H), 1.00 (3H), 0.85 (3H)

### Synthesis of Compound (3d) (Example 4, formula (1-4))

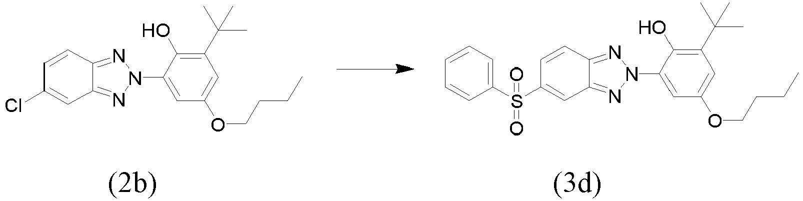

The synthesis method of Compound (3d) is similar to those of Compound (1c) and Compound (1d). The starting material used herein was 40.0 g of Compound (2b) and 35.4 g of yellow solids were obtained (mp = 148.3°C).
HRMS ESI [M+H]⁺: calc. 480.1957, found 480.1964
¹H NMR (400MHz, CDCl₃): δ 11.21 (1H), 8.71 (1H), 7.95-8.05 (3H), 7.90 (1H), 7.77 (1H), 7.40-7.70 (3H), 7.06 (1H), 4.03 (2H), 1.81 (2H), 1.54 (2H), 1.48 (9H), 1.00 (3H)

### Synthesis of raw material Compound (3a)

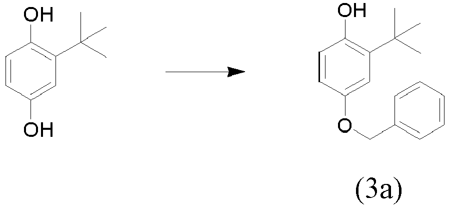

The synthesis method of Compound (3a) is similar to that of Compound (1a). The starting material used herein was 856.8 g of tertiary butylhydroquinone and 960.0 g of benzyl bromide, and 670.0 g of yellow liquid was obtained.

### Synthesis of Compound (4b)

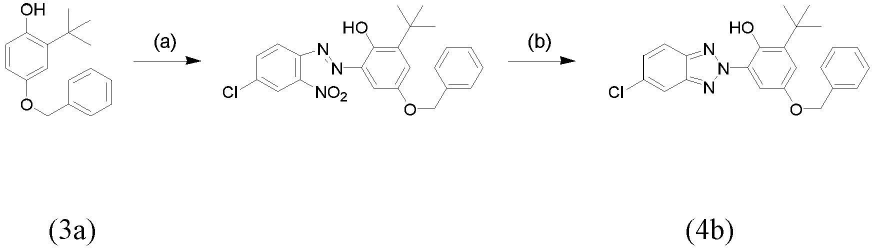

The synthesis method of Compound (4b) is similar to that of Compound (1b). The starting material used herein was 450.0 g of the raw material Compound (3a), and 138.0 g of yellow solids were obtained (mp = 130.7°C).
HRMS ESI [M-H]⁻: calc. 406.1322, found 406.1324
¹H NMR (400MHz, CDCl₃): δ 11.36 (1H), 7.80-8.00 (3H), 7.49 (2H), 7.30-7.45 (4H), 7.10 (1H), 5.11 (2H), 1.48 (9H)

### Synthesis of Compound (4d) (Example 5, formula (I-5))

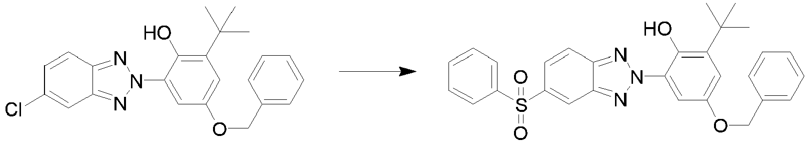

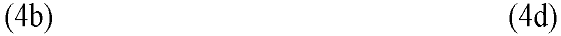

The synthesis method of Compound (4d) is similar to those of Compound (1c) and Compound (1d). The starting material used herein was 40.0 g of Compound (4b), and 34.7 g of yellow solids were obtained (mp = 157.4°C).
HRMS ESI [M+H]⁺: calc. 514.1801, found 514.1807
¹H NMR (400MHz, CDCl₃): δ 11.25 (1H), 8.71 (1H), 7.95-8.05 (3H), 7.80-7.95 (2H), 7.30-7.70 (8H) 7.13 (1H), 5.12 (2H), 1.48 (9H)

### Synthesis of Compound (5b) (Comparative example 6, formula (1-6))

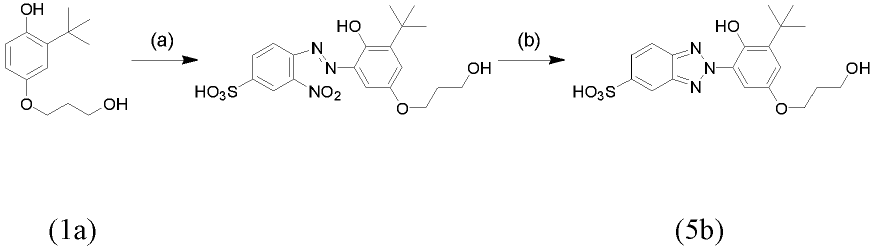

### Step (a)

The synthesis method of Compound (1b) was used herein, and the starting material was 128.5 g of 4-sulfo-2-nitroaniline and 120.0 of Compound (1a). The product was directly used in the next step without purification.

### Step (b)

The azo dye obtained in the step (a) was dissolved in 300 mL isopropanol and 300 mL of water. 30 g of NaOH was added into the solution at 0°C, followed by slowly adding 60 g of formamidinesulfinic acid. After reacting at 80°C for 6 hr, the temperature of the reaction solution was back to room temperature. Then, 70 mL of HCl was used to adjust the reaction solution to neutral. 200 mL of the solvent was removed with reduced pressure distillation, the solid salt was filtered out. After extracting and washing with 100 mL of toluene, the solution was acidified with 50 mL of HCl at 0°C, followed by recrystallization with 1 g of NaCl. The solids were filtered and collected at 0°C, and the surfaces of the solids were washed with cold ethanol. After vacuum drying, 67.8 g of yellow solids were obtained (mp = 243.0°C).
HRMS ESI [M-H]⁻: calc. 420.1229, found 420.1243
¹H NMR (400MHz, CD₃OD): δ 8.44 (1H), 8.03 (1H), 7.97 (1H), 7.79 (1H), 7.00 (1H), 4.12 (2H), 3.78 (2H), 2.04 (2H), 1.49 (9H)

### Synthesis of Compound (6b)

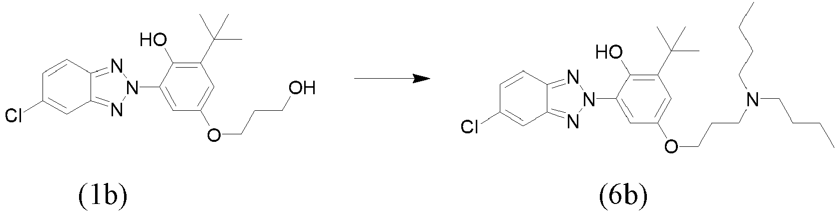

7.52 g of Compound (1b) was dissolved in 20 mL of dichloromethane, followed by adding 1.38 g of imidazole and 5.25 g of triphenylphosphine. After all dissolved, the solution was cooled down to 0°C. After adding 5.10 g of iodine, the temperature of the solution was back to room temperature and stirred for 4 hr. Then, 10 mL of saturated Na₂SO₃ solution was added, and then 20 mL of 0.1 N HCl was added. After drying and concentration, 10 mL of acetonitrile and 1.62 g of di-n-butylamine was added. After stirring at 50°C for 2 hr, 20 mL of tulene and 20 mL of 0.1 N HCl was added for extraction, followed by reduced pressure distillation to obtain 6.8 g of yellow oil.
HRMS ESI [M+H]⁺: calc. 487.2840, found 487.2850
¹H NMR (400MHz, CD₃OD): δ 11.32 (1H), 7.91 (1H), 7.86 (1H), 7.77 (1H), 7.42 (1H), 7.02 (1H), 4.08 (2H), 2.62 (2H), 2.43 (4H), 1.94 (2H), 1.49 (9H), 1.42 (4H), 1.30 (4H), 1.91 (6H)

### Synthesis of Compound (6c) (Example 7, formula (1-7))

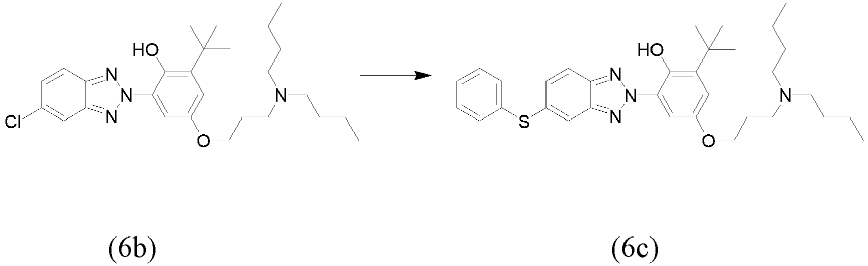

The synthesis method of Compound (6c) is similar to that of Compound (1c). The starting material used herein was 3.0 g of Compound (6b), and 2.92 g of yellow oil was obtained.
HRMS ESI [M+H]⁺: calc. 561.3263, found 561.3267
¹H NMR (400MHz, CDCl₃): δ 11.39 (1H), 7.81 (1H), 7.75 (1H), 7.68 (1H), 7.45-7.50 (2H), 7.25-7.41 (4H), 7.00 (1H), 4.06 (2H), 2.62 (2H), 2.43 (4H), 1.94 (2H), 1.48 (9H), 1.43 (4H), 1.30 (4H), 1.91 (6H)

### Synthesis of Compound (7b)

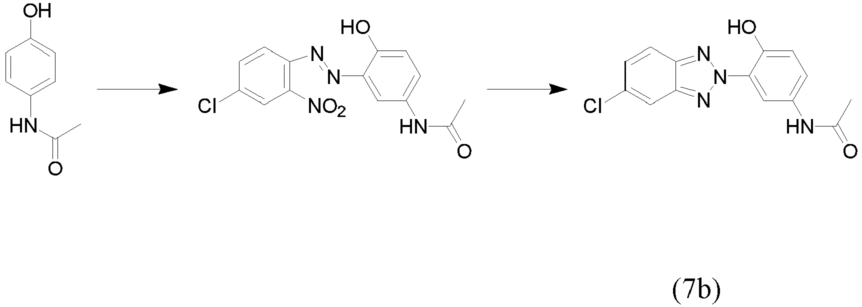

The synthesis method of Compound (7b) is similar to that of Compound (1b). The starting material used herein was 500.0 g of acetaminophen, and 110.2 g of white solids were obtained.
HRMS ESI [M-H]⁻: calc. 301.0492, found 301.0500
¹H NMR (400MHz, d6-DMSO): δ 10.30 (1H), 10.05 (1H), 8.24 (2H), 8.11 (1H), 7.55 (1H), 7.50 (1H), 7.12 (1H), 2.05 (3H)

### Synthesis of Compound (7d) (Example 8, formula (1-8))

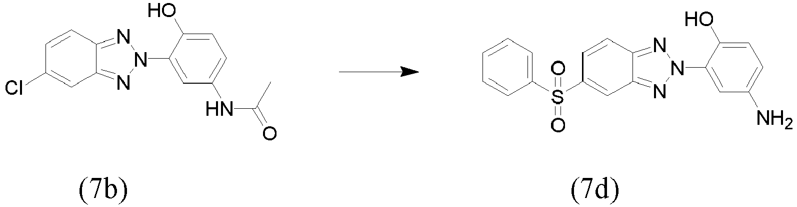

The synthesis method of Compound (7d) is similar to those of Compound (1c) and Compound (1d). The starting material used herein was 40.0 g of Compound (7b) to obtaine 41.5 g of an acetyl-protected intermediate. During the step of removing the acetyl protection, 6.5 g of NaOH was added and the reaction solution was refluexed in 100 mL of ethanol for 5 hr. 1 N HCl was used to adjust the pH of the reaction solution to neutral, and the reaction solution was added into ice for filtration. Finally, 33.9 g of orange solids were obtained (mp = 188.0°C). HRMS ESI [M+H]⁺: calc. 367.0865, found 367.0867
¹H NMR (400MHz, CDCl₃): δ 10.36 (1H), 8.72 (1H), 8.00-8.04 (3H), 8.89 (1H), 7.70 (1H), 7.50-7.63 (3H), 7.04 (1H), 6.79 (1H), 3.66 (2H)

### Synthesis of Compound (8d) (Example 9, formula (I-9))

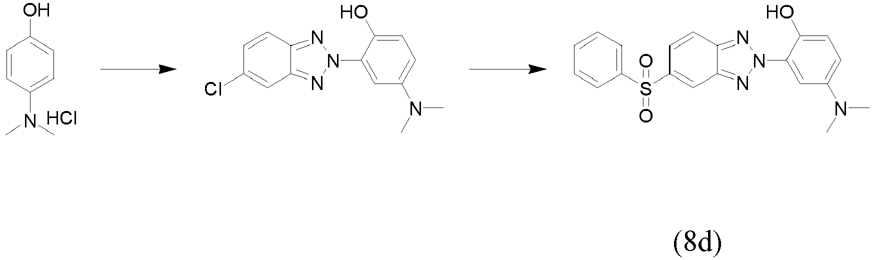

The synthesis method of Compound (8d) is similar to those of Compounds (1b) to (1d). The starting material used herein was 500.0 g of 4-(dimethylamino)phenol, and 81.1 g of orange solids were obtained (mp = 175.8°C).
HRMS ESI [M+H]⁺: calc. 395.1178, found 395.1177
¹H NMR (400MHz, CDCl₃): δ 10.35 (1H), 8.72 (1H), 8.00-8.04 (3H), 7.89 (1H), 7.70 (1H), 7.50-7.61 (3H) 7.11 (1H), 6.90 (1H), 2.99 (6H)

### Synthesis of Compound (9d) (Example 10, formula (I-10))

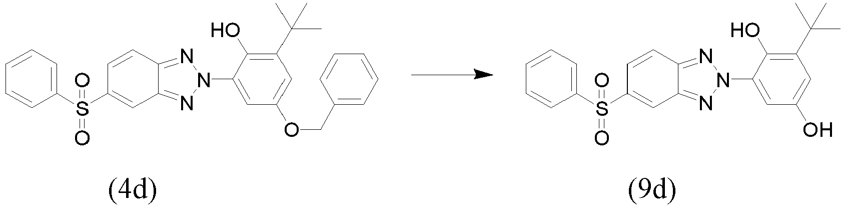

25.6 g of Compound (4d) was placed in an autoclave and dissolved in 300 mL of toluene. 0.26 g of palladium on carbon (10%) was added into the reaction solution. 200 psi of H₂ was introduced. The reaction solution was stirred at 50°C for 6 hr. After the temperature of the reaction solution was back to room temperature, the reaction solution was filtered and recrystallized with 500 mL of cold methanol to obtain 18.6 g of yellow solids (mp = 151.4°C).
HRMS ESI [M+H]⁺: calc. 424.1326, found 424.1345
¹H NMR (400MHz, CDCl₃): δ 11.14 (1H), 8.70 (1H), 7.98-8.05 (3H), 7.89 (1H), 7.77 (1H), 7.50-7.63 (3H), 7.00 (1H), 4.70 (1H), 1.47 (9H)

### Synthesis of Compound (10b) (Comparative Example 1)

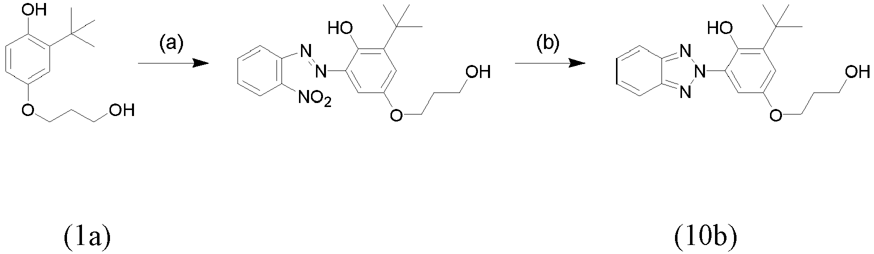

The synthesis method of Compound (10b) is similar to that of Compound (1b). The starting material used herein was 73.2 g of 2-nitroaniline and 112.2 g of Compound (1a), and 67.8 g of yellow solids were obtained (mp = 140.6°C).
HRMS ESI [M+H]⁺: calc. 342.1818, found 342.1821
¹H NMR (400MHz, DMSO-d6): δ 11.04 (1H), 8.08 (2H), 7.55-7.65 (3H), 6.96 (1H), 4.58 (1H), 4.08 (2H), 3.58 (2H), 1.90 (2H), 1.45 (9H) TGA (5% weight loss): 250.01°C

### Synthesis of Compound (11c) (Comparative Example 2)

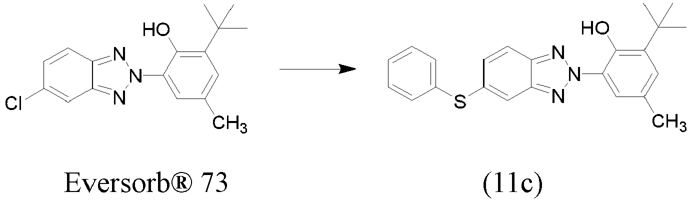

The synthesis method of Compound (11c) is similar to that of Compound (1c). The starting material used herein was 10 g of Eversorb^{®} 73, and 11.5 g of white solids were obtained (mp = 134.0°C). HRMS ESI [M+H]⁺: calc. 390.1640, found 390.1631
¹H NMR (400MHz, CDCl₃): δ 11.54 (1H), 8.02 (1H), 7.81 (1H), 7.68 (1H), 7.45-7.55 (2H), 7.30-7.45 (4H), 7.16 (1H), 2.37 (3H), 1.48 (9H) TGA (5% weight loss): 269.79°C

### Synthesis of Compound (11d) (Comparative Example 3)

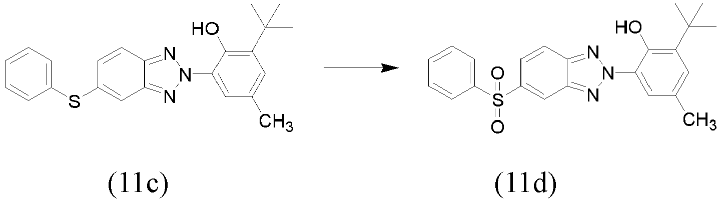

The synthesis method of Compound (11d) is similar to that of Compound (1d). The starting material, Compound (1b), used in the synthesis of Compound (1d) was changed to 5.3 g of Compound (11c), and 4.7 g of light yellow solids were obtained (mp = 142.5°C).
HRMS ESI [M+H]⁺: calc. 422.1538, found 422.1531
¹H NMR (400MHz, DMSO-d6): δ 10.55 (1H), 8.85 (1H), 8.27 (1H), 8.00-8.15 (2H), 7.97 (1H), 7.60-7.80 (4H), 7.25 (1H), 2.32 (3H), 1.44 (9H)
TGA (5% weight loss): 309.50°C

### Comparative Example 4: Tinuvin^{®} CarboProtect^{®} from BASF Co.

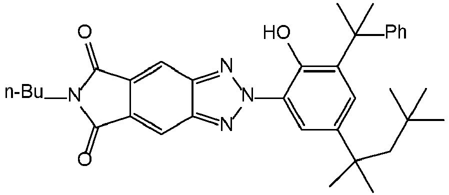

### Comparative Example 5: Eversorb^{®} 73 from Everlight Chemical

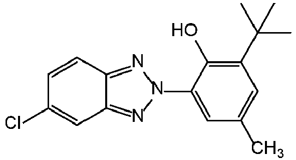

### Testing Example 1: Comparisons of UV absorption spectra

The benzotriazole compounds of Examples 1 to 9 and Comparative Examples 1 to 3 and the UV absorbers of Comparative Examples 4 and 5 were formulated into 20 ppm solution (methanol:THF=90:10). Then, the absorbance of the solutions were measured with an UV-visible spectrophotometer (UV-2600; Shimadzu Instruments Co., Ltd.), and the results are listed in the following Table 1, wherein the maximum absorption wavelength (λₘₐₓ) is the absorption wavelength of the absorption peak (nm) in the long-wavelength region, and the effective absorption limit is the absorption wavelength (nm) that the absorbance is 0.05.

**Table 1**

| Sample | Molecular weight | Absorption area at 290-340nm | Absorption area at 380-400nm | Absorption area at 400-480nm | λₘₐₓ (nm) | effective absorption limit (nm) (absorbance = 0.05) |
|---|---|---|---|---|---|---|
| Example 1 | 449.6 | 22.6 | 15.6 | 7.4 | 374 | 428 |
| Example 2 | 481.6 | 28.8 | 10.5 | 10.7 | 382 | 444 |
| Example 3 | 571.8 | 21.3 | 7.6 | 7.7 | 382 | 440 |
| Example 4 | 479.6 | 28.9 | 10.2 | 10.7 | 383 | 446 |
| Example 5 | 513.6 | 26.3 | 8.9 | 8.7 | 381 | 441 |
| Comparative example 6 | 421.5 | 23.4 | 6.6 | 2.9 | 366 | 421 |
| Example 8 | 366.4 | 34.6 | 6.2 | 18.3 | 401 | 489 |
| Example 9 | 394.4 | 44.5 | 4.7 | 21.2 | 420 | 511 |
| Comparative Example 1 | 341.4 | 33.7 | 7.6 | 1.8 | 359 | 414 |
| Comparative Example 2 | 389.5 | 27.6 | 10.3 | 0.8 | 363 | 405 |
| Comparative Example 3 | 421.5 | 30.8 | 6.9 | 1.0 | 360 | 411 |
| Comparative Example 4 | 566.7 | 18.3 | 7.8 | 3.4 | 371 | 420 |
| Comparative Example 5 | 315.8 | 51.0 | 4.2 | 0 | 349 | 399 |

From the results shown in Table 1, the effective absorption limits of the compounds of Examples 1 to 9 can reach to 420 nm and have broader absorption ranges, and the compounds of Examples 1 to 9 have the absorption peaks that the commercial UV absorbers have in the region with wavelengths from 290 nm to 340 nm. In addition, the effective absorption limit of the compound of Example 9 can be farthest extended to 500 nm or more. Compared to the compound of Comparative Example 5, the compounds of Comparative Examples 1 to 3 with the O-containing group on the phenyl ring or the S-containing group on the benzo ring have slight red shift on the absorption spectra thereof. However, there is no collaboration between the O-containing group on the phenyl ring and the S-containing group on the benzo ring, so the maximum absorption wavelengths of the compounds of Comparative Examples 1 to 3 are less than the maximum absorption wavelengths of the compounds of Examples 1 to 9.

### Texting Example 2: Transmittance test

0.2 g of the benzotriazole UV absorbers prepared by Exmaples 1 and 2 and Comparative Examples 1 to 3 and the UV absorbers of Comparative Example 5 were respectively dissolved in 10 g of a mixing solvent (ethylene glycol monomethyl ether acetate:toluene:n-butyl acetate:ethyl acetate=20:50:20:10, which was prepared by the weight ratio). Transparent solutions containing UV absorbers were obtained after stirring at room temperature. 20 g of polyurethane (product model: A-7121) as a main component and 10 g of hardener (product model: Bayer N75) were mixed in advance, followed by stirring at room temperature to obtain a two-component polyurethane varnish (2K PU). The aforsaid transparent solutions were respectively added into the 2K PU varnish. After stirring at room temperature, transparent varnishes containing UV absorbers were obtained.

Glass substrates were placed on a coating machine (KCC101; RK Print-Coat Instruments Ltd.), and the aforesaid transparent varnished were applied onto surfaces of the glass substrates with spreading rods (Rod type #18) to form wet films having thicknesses of about 40 µm. After drying at room temperature for 8 hr, glass substrates coated with transparent coating layers containing benzotriazole UV absorbers were obtained. Next, an UV-visible spectrophotometer (UV-2600; Shimadzu Instruments Co., Ltd.) was used to measure the transmittance (T%) of the obtained glass substrates. The results are listed in the following Table 2, in which light cut-off wavelength (transmittance = 99%) and the transmittance measured at 420 nm were listed.

**Table 2: Measurement results of the transmittances of the glass substrates coated with transparent coating layers**

| Sample | Molecular weight | Light cut-off wavelength (nm) | Transmittance (measured at 420 nm) |
|---|---|---|---|
| Example 1 | 449.6 | 434 | 67% |
| Example 2 | 481.6 | 450 | 38% |
| Comparative Example 1 | 341.4 | 422 | 90% |
| Comparative Example 2 | 389.5 | 414 | 100% |
| Comparative Example 3 | 421.5 | 422 | 94% |
| Comparative Example 5 | 315.8 | 404 | 100% |
| Blank | - | - | 100% |

From the results shown in Table 2, the transmittances of the compounds of Examples 1 and 2 at 420 nm were much less than the transmittances of the compounds of Comparative Examples 1 to 3. These results indicate that the abosrption of the compounds of Examples 1 and 2 have red shift.

In conclusion, the absorption spectrum of the novel compound provided by the present disclosure shows red shift. When the novel compound of the present disclosure is applied to a substrate sensitive to an electro-magnetic radiation having wavelengths greater than 380 nm to form a coating layer, the coating layer can effectively absorb the light having wavelength greater than 380 nm. Hence, the novel compound provided by the present disclosure can be used to form a blue light blocking coating layer or an anti-UV coating layer, to provide a product having blue light blocking effect or anti-UV effect such as a glasses lens or a screen protector.

## Claims

1. A compound represented by the following formula (I): 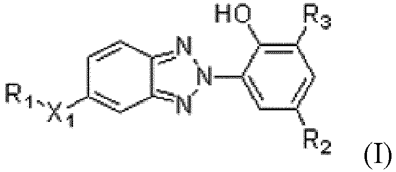
wherein X₁ is -S-, -SO-, or -SO₂-;
R₁ is C₁₋₂₄ linear or branched alkyl or C₆₋₁₅ aryl;
R₂ is -OR₄ or -NR₅R₆, wherein R₄, R₅ and R₆ respectively are H, C₁₋₁₀ linear or branched alkyl, C₇₋₁₅ phenylalkyl, or C₁₋₁₀ linear or branched alkyl substituted by one or more -OH or -NR₁₁R₁₂; R₁₁ is H or C₁₋₁₀ linear or branched alkyl; R₁₂ is H, C₁₋₁₀ linear or branched alkyl or -C(=O)R₁₈, wherein R₁₈ is linear or branched C₂₋₁₀ alkenyl, -OR₁₉ or -NR₂₀R₂₁, R₁₉ is C₁₋₁₀ linear or branched alkyl or C₆₋₁₅ aryl, and R₂₀ and R₂₁ respectively are H, C₁₋₁₀ linear or branched alkyl or C₆₋₁₅ aryl; and
R₃ is H or C₁₋₁₀ linear or branched alkyl,
when R₂ is -OR₄, R₄ is H, C₁₋₁₀ linear or branched alkyl, C₇₋₁₅ phenylalkyl or C₁₋₁₀ linear or branched alkyl substituted by one or more -OH or -NR₁₁R₁₂ and R₃ is C₁₋₁₀ linear or branched alkyl; and
when R₂ is -NR₅R₆, R₅ and R₆ respectively are H or C₁₋₁₀ linear or branched alkyl and R₃ is H.

2. The compound of claim 1, wherein R₁ is C₁₋₁₅ linear or branched alkyl or C₆₋₁₅ aryl, preferably wherein R₁ is C₁₋₁₅ linear or branched alkyl, phenyl or naphthyl.

3. The compound of claim 1 or 2, wherein R₂ is -OR₄.

4. The compound of claim 3, wherein R₁₂ is H or C₁₋₁₀ linear or branched alkyl.

5. The compound of claim 1 or 2, wherein R₂ is -NR₅R₆.

6. The compound of any of the previous claims 1 to 4, wherein R₃ is tert-butyl.

7. The compound of claim 1, which is anyone represented by the following formulas (I-1) to (I-5) and (I-7) to (I-10):

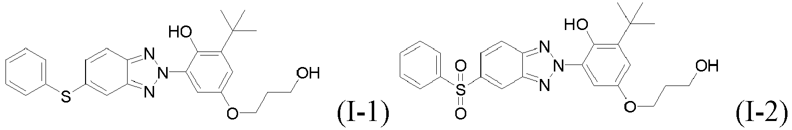

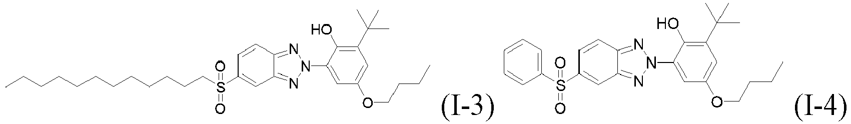

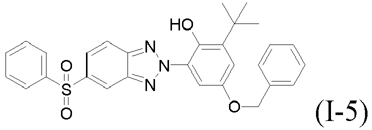

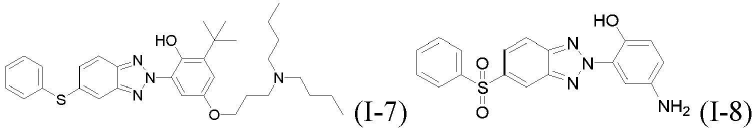

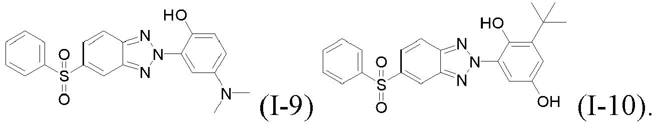

8. A coating layer formed on a substrate, wherein the coating layer comprises a compound according to any of the claims 1 to 7, wherein the substrate is sensitive to an electro-magnetic radiation having wavelengths greater than 380 nm.

9. A lens for a blue light blocking glasses, comprising:
a compound according to any of the claims 1 to 7.

10. An anti-blue light screen protector, comprising:
a compound according to any of the claims 1 to 7.

## Patentansprüche

1. Verbindung, dargestellt durch die folgende Formel (I) : 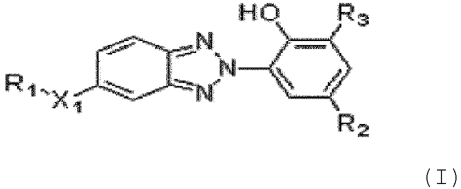
wobei X₁ -S-, -SO- oder -SO₂- ist;
R₁ ein lineares oder verzweigtes C₁₋₂₄ Alkyl oder C₆₋₁₅ Aryl ist;
R₂ -OR₄ oder -NR₅R₆ ist, wobei R₄, R₅ und R₆ jeweils H, ein lineares oder verzweigtes C₁₋₁₀ Alkyl, C₇₋₁₅ Phenylalkyl oder lineares oder verzweigtes C₁₋₁₀ Alkyl, substituiert durch ein oder mehrere -OH oder -NR₁₁R₁₂ sind; R₁₁ H oder ein lineares oder verzweigtes C₁₋₁₀ Alkyl ist; R₁₂ H, ein lineares oder verzweigtes C₁₋₁₀ Alkyl oder -C(=O)R₁₈ ist,
wobei R₁₈ ein lineares oder verzweigtes C₂₋₁₀ Alkenyl, - OR₁₉ oder -NR₂₀R₂₁ ist, R₁₉ ein lineares oder verzweigtes C₁₋₁₀ Alkyl oder C₆₋₁₅ Aryl ist, und R₂₀ und R₂₁ jeweils H, ein lineares oder verzweigtes C₁₋₁₀ Alkyl oder C₆₋₁₅ Aryl sind; und
R₃ H oder ein lineares oder verzweigtes C₁₋₁₀ Alkyl ist, wenn R₂ -OR₄ ist, ist R₄ H, ein lineares oder verzweigtes C₁₋₁₀ Alkyl, C₇₋₁₅ Phenylalkyl oder lineares oder verzweigtes C₁₋₁₀ Alkyl, substituiert durch ein oder mehrere -OH oder -NR₁₁R₁₂ und R₃ ist ein lineares oder verzweigtes C₁₋₁₀ Alkyl; und
wenn R₂ -NR₅R₆ ist, sind R₅ und R₆ jeweils H oder ein lineares oder verzweigtes C₁₋₁₀ Alkyl und R₃ ist H.

2. Verbindung nach Anspruch 1, wobei R₁ ein lineares oder verzweigtes C₁₋₁₅ Alkyl oder C₆₋₁₅ Aryl ist, vorzugsweise wobei R₁ ein lineares oder verzweigtes C₁₋₁₅ Alkyl, Phenyl oder Naphthyl ist.

3. Verbindung nach Anspruch 1 oder 2, wobei R₂ -OR₄ ist.

4. Verbindung nach Anspruch 3, wobei R₁₂ H oder ein lineares oder verzweigtes C₁₋₁₀ Alkyl ist.

5. Verbindung nach Anspruch 1 oder 2, wobei R₂ -NR₅R₆ ist.

6. Verbindung nach einem der vorhergehenden Ansprüche 1 bis 4, wobei R₃ tert-Butyl ist.

7. Verbindung nach Anspruch 1, die durch jedwede der folgenden Formeln (I-1) bis (1-5) und (1-7) bis (1-10) dargestellt wird:

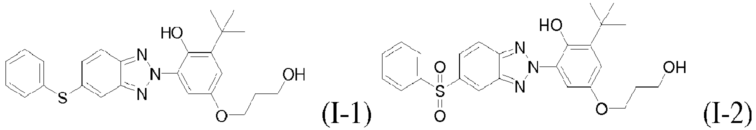

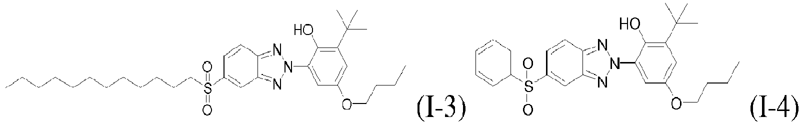

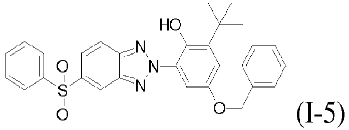

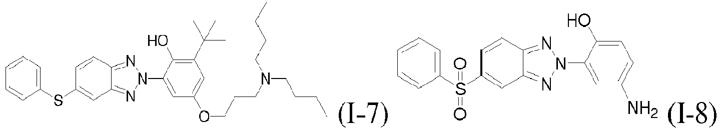

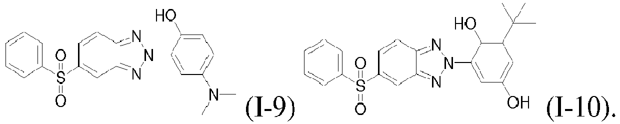

8. Auf einem Substrat gebildete Überzugsschicht, wobei die Überzugsschicht eine Verbindung nach einem der Ansprüche 1 bis 7 umfasst, wobei das Substrat für eine elektromagnetische Strahlung mit Wellenlängen von mehr als 380 nm empfindlich ist.

9. Linse für eine blaues Licht blockierende Brille, umfassend:
eine Verbindung nach einem der Ansprüche 1 bis 7.

10. Bildschirmschutz gegen blaues Licht, umfassend:
eine Verbindung nach einem der Ansprüche 1 bis 7.

## Revendications

1. Composé représenté par la formule (I) suivante : 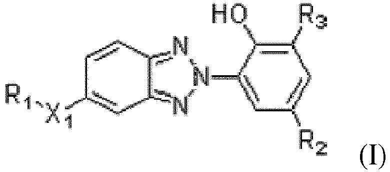
dans lequel X₁ est -S-, -SO- ou -SO₂- ;
R₁ est un alkyle en C₁ à C₂₄ linéaire ou ramifié ou un aryle en C₆ à C₁₅ ;
R₂ est -OR₄ ou -NR₅R₆, où R₄, R₅ et R₆ sont respectivement H, un alkyle en C₁ à C₁₀ linéaire ou ramifié, un phénylalkyle en C₇ à C₁₅, ou un alkyle en C₁ à C₁₀ linéaire ou ramifié substitué par un ou plusieurs -OH ou -NR₁₁R₁₂ ; R₁₁ est H ou un alkyle en C₁ à C₁₀ linéaire ou ramifié ; R₁₂ est H, un alkyle en C₁ à C₁₀ linéaire ou ramifié ou -C(=O)R₁₈, où R₁₈ est un alcényle en C₂ à C₁₀ linéaire ou ramifié, -OR₁₉ ou -NR₂₀R₂₁, R₁₉ est un alkyle en C₁ à C₁₀ linéaire ou ramifié ou un aryle en C₆ ou C₁₅, et R₂₀ et R₂₁ sont respectivement H, un alkyle en C₁ à C₁₀ linéaire ou ramifié ou un aryle en C₆ à C₁₅ ; et
R₃ est H ou un alkyle en C₁ à C₁₀ linéaire ou ramifié, quand R₂ est -OR₄, R₄ est H, un alkyle en C₁ à C₁₀ linéaire ou ramifié, un phénylalkyle en C₇ à C₁₅ ou un alkyle en C₁ à C₁₀ linéaire ou ramifié substitué par un ou plusieurs -OH ou -NR₁₁R₁₂ et R₃ est un alkyle en C₁ à C₁₀ linéaire ou ramifié ; et
quand R₂ est -NR₅R₆, R₅ et R₆ sont respectivement H ou un alkyle en C₁ à C₁₀ linéaire ou ramifié et R₃ est H.

2. Composé selon la revendication 1, dans lequel R₁ est un alkyle en C₁ à C₁₅ linéaire ou ramifié ou un aryle en C₆ à C₁₅, de préférence dans lequel R₁ est un alkyle en C₁ à C₁₅ linéaire ou ramifié, phényle ou naphtyle.

3. Composé selon la revendication 1 ou 2, dans lequel R₂ est -OR₄.

4. Composé selon la revendication 3, dans lequel R₁₂ est H ou un alkyle en C₁ à C₁₀ linéaire ou ramifié.

5. Composé selon la revendication 1 ou 2, dans lequel R₂ est -NR₅R₆.

6. Composé selon l'une quelconque des revendications précédentes 1 à 4, dans lequel R₃ est le tert-butyle.

7. Composé selon la revendication 1, qui est l'un quelconque représenté par les formules suivantes (I-1) à (1-5) et (1-7) à (1-10) :

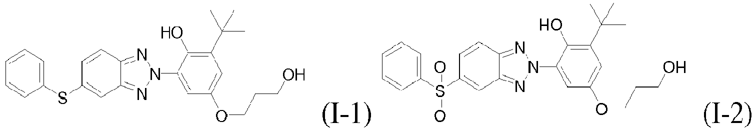

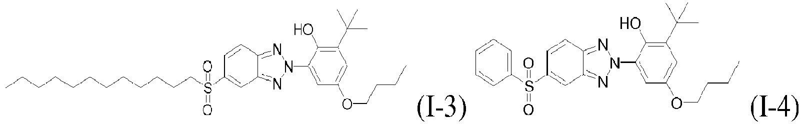

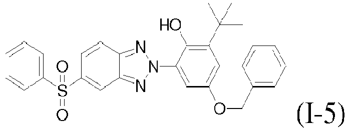

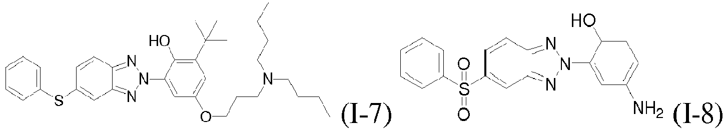

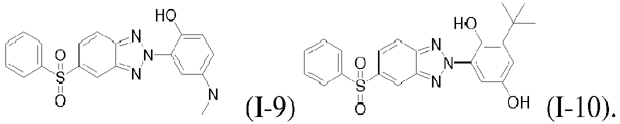

8. Couche de revêtement formée sur un substrat, où la couche de revêtement comprend un composé selon l'une quelconque des revendications 1 à 7, dans laquelle le substrat est sensible à un rayonnement électromagnétique ayant des longueurs d'onde supérieures à 380 nm.

9. Verres pour lunettes bloquant la lumière bleue, comprenant :
un composé selon l'une quelconque des revendications 1 à 7.

10. Protecteur d'écran anti-lumière bleue, comprenant :
un composé selon l'une quelconque des revendications 1 à 7.
